Europäisches Patentamt

(19) European Patent Office

Office européen des brevets

(11) Publication number: **0 069 236**
**B1**

(12) **EUROPEAN PATENT SPECIFICATION**

(45) Date of publication of patent specification: **06.11.85**

(21) Application number: **82104971.5**

(22) Date of filing: **07.06.82**

(51) Int. Cl.⁴: **A 61 M 1/32**

(54) **A device for the transfer of one or more substances between a gas and a liquid.**

(30) Priority: **07.07.81 SE 8104219**

(43) Date of publication of application:
**12.01.83 Bulletin 83/02**

(45) Publication of the grant of the patent:
**06.11.85 Bulletin 85/45**

(84) Designated Contracting States:
**AT BE CH DE FR GB IT LI LU NL**

(56) References cited:
**EP-A-0 035 266**
**US-A-4 158 693**
**US-A-4 261 951**

(73) Proprietor: **GAMBRO CARDIO AB**
**Box 10101**
**S-220 10 Lund (SE)**

(72) Inventor: **Johnsson, Bo**
**Stormgatan 39A**
**S-261 37 Landskrona (SE)**
Inventor: **Losell, Ernst Ingvar**
**Cymbalvägen 20**
**S-245 00 Staffanstorp (SE)**
Inventor: **Palmqvist, Sune Haakon**
**Nordstrands väg 19**
**S-245 00 Staffanstorp (SE)**
Inventor: **Stenberg, Kaj Ove**
**Fugavägen 53**
**S-245 00 Staffanstorp (SE)**

(74) Representative: **Boberg, Nils Gunnar Erik**
**Gambro AB Patent Department Box 10101**
**S-220 10 Lund (SE)**

Courier Press, Leamington Spa, England.

## Description

Technical Field

The present invention relates to a device for the transfer of one or more substances between a gas and a liquid, the gas being introduced into the liquid in the form of bubbles, from which the said substances are transferred to the fluid, whereafter the excess gas is removed together with any substances transferred from the liquid to the gas.

The device in accordance with the invention is intended in the first place to be used for the oxygenation of blood, that is to say as an oxygenator. It is clear, however, to those versed in the art that the construction in accordance with the invention can also be used for many other purposes, that is to say substantially wherever substances from a gas are to be made to react with or to dissolve in a liquid or else if the substances are to be exchanged between gases and liquids.

Background Art

Insofar as oxygenators are concerned, three main types can be distinguished. The oldest is probably the type where a blood film is formed in direct contact with an atmosphere containing oxygen. In a second type the oxygen is made instead to diffuse through a semipermeable membrane from an atmosphere containing oxygen on the one side of the membrane to the blood on the other side. The third type, to which belongs the device in accordance with the invention, is known under the name of bubble oxygenator. In this type the gas bubbles containing oxygen are introduced directly into the blood so as directly to act on the same. After the desired action the excess gas is removed together with carbon dioxide withdrawn from the blood.

Examples of constructions of the last mentioned type, that is to say of bubble oxygenators, are shown for example in the following US patent specifications: 3 175 555, 2 934 067, 3 545 937, 4 073 622, 3 468 631, 3 488 158, 3 615 238, 4 058 369, 3 578 411, 3 291 568, 4 033 724, 3 827 860, 3 853 479, 4 067 696, 4 065 264, 4 138 464, 4 138 288.

More particularly, the present invention constitutes a further development of the device according to EP—A—0 035 266, published after the priority date of the present application.

Reference is finally made to US—A—4 261 951 describing an oxygenator essentially corresponding to the preamble of our following main claim and US—A—4 158 693 describing a heat exchanger similar to the one used in a preferred embodiment of the present invention.

Disclosure of the Invention

The device in accordance with the invention comprises an annular heat and gas exchange chamber designed for vertical placing and having a gas inlet at the lower end of this chamber and a heat exchanger arranged above this inlet and wherein the heat and gas exchange chamber is terminated at the top by a number of ports distributed evenly around the periphery, and is characterized in that the heat and gas exchange chamber is formed between an inner gas and liquid inlet pipe and a vessel arranged outside the same which is open at the top, the ports being constituted of recesses in the top edge of this vessel, which is preferably arranged so as to be in contact against a lid provided over the vessel and the said pipe. Through this arrangement a very uniform and homogeneous flow around the whole periphery of the heat and gas exchange chamber is achieved. In this manner a very good heat transfer or gas exchange is obtained.

To prevent any damage to the blood when the device is used as an oxygenator in connection with the throttling, the ports may be approximately rounded off evenly by a double-curved shape.

In a preferred embodiment of the invention, the device comprises an outer casing arranged outside the said vessel, which between itself and the vessel forms a skimming chamber with a filter for the final breaking down of the gas bubbles. This filter is appropriately arranged obliquely so that between the said vessel and the filter a wedge-shaped collecting space for the liquid widening upwards is formed, into which open the said ports. This construction has proved to be suitable from a point of view of assembly as well as from a point of view of efficiency.

The heat exchanger is suitably shorter than the heat and gas exchange chamber and may be separated from the gas inlet by means of a mixing chamber. By this arrangement the bubbles can be formed in this mixing chamber irrespectively of the design of the heat exchanger.

The gas inlet is preferably constituted of a plurality of holes of substantially the same size distributed evenly around the periphery of the chamber for the formation of bubbles which are partly broken down in a plurality of gaps formed by the heat exchanger in the heat and gas exchange chamber, to be broken down further, subsequently, in the said ports.

It should be mentioned that the transfer of carbon dioxide is normally considered to be improved by large bubbles whilst the transfer of oxygen is considered to be at its most effective by means of small bubbles.

The heat exchanger may consist of two pipe parts coiled in helical manner, one of which is coiled outside the other, these two parts being separated by a first gap and forming a second and a third gap, respectively between themselves and the inner and outer wall respectively of the annular heat and gas exchange chamber. The two parts of the heat exchanger coiled in helical manner suitably constitute parts of one and the same pipe, whose ends are threaded through the lid provided above the heat and gas exchange chamber. It has been found that this heat exchanger gives a very good heat transfer, at the same time as it facilitates the mixing process owing to the gas bubbles being partly transformed and/or broken down. At the same time the construction is completely free of leakage problems and is very suitable from a point of view of installation.

As mentioned already, the construction in accordance with the invention is used chiefly as a so-called oxygenator, the said liquid being constituted of blood and the said gas being constituted of oxygen or an oxygen mixture from which oxygen is transferred to the blood and to which carbon dioxide is transferred from the blood. The invention is described, therefore, in the following with reference to such a construction, made up of a number of concentric vessels arranged concentrically inside one another which can easily be sealed off in relation to each other.

Brief Description of Drawings

Fig. 1 shows a longitudinal section through an oxygenator which falls outside the scope of the present invention but may easily be modified in accordance with the invention.

Figs. 2 and 3 show a side view and an end view respectively of a heat exchanger forming part of the oxygenator.

Fig. 4 shows a longitudinal section through an intermediate vessel forming part of an oxygenator according to the present invention.

Fig. 5 shows an end view of the corresponding vessel seen from the open upper end of the same.

Figs. 6 and 7 finally show a section along the line VI—VI and a section perpendicular thereto through a throttling port provided at the top edge of the vessel shown in Fig. 4 and 5, respectively.

Best Mode of Carrying Out the Invention

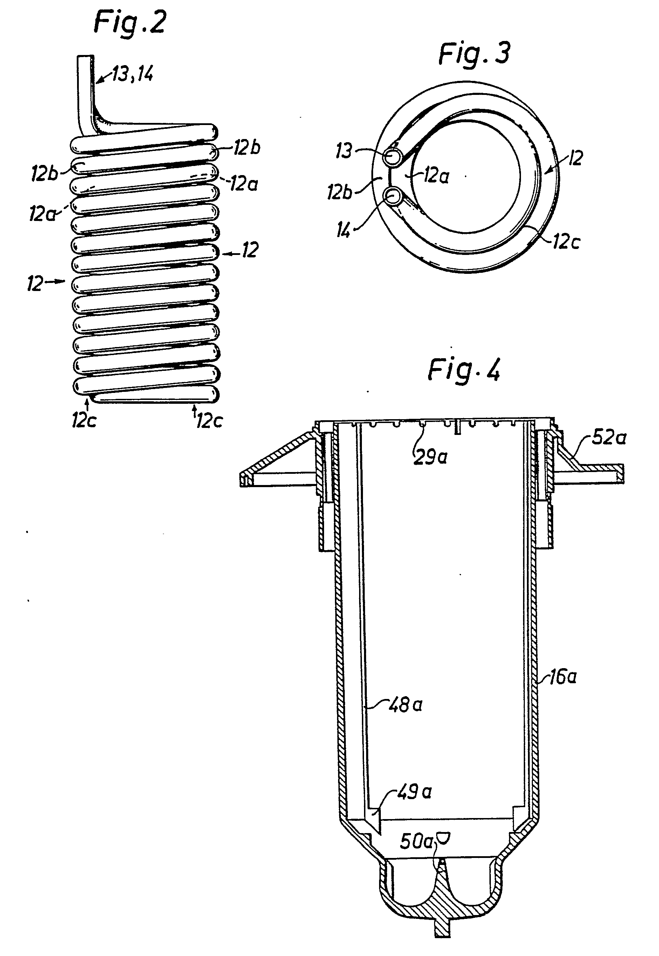

In the embodiment shown as an example venous blood is supplied from a patient to the inlet nozzle 1 arranged at the top. This nozzle together with a second inlet nozzle 2 and a sampling nozzle 3 is arranged on a branch pipe 4 which is freely rotatable in respect of the rest of the oxygenator. This branch pipe 4 is threaded onto a double-walled inlet pipe 5 arranged centrally in the oxygenator which contains an inner central liquid inlet duct 6 and an outer annular gap 7 for the gas supply. Between the inlet pipe 5 and the branch pipe 4 a packing 8 is provided. Thus the blood is caused to flow vertically down through the duct 6 and via a smooth passage 9 into a narrow annular gap 10. This gap subsequently passes into a widened annular vertical cylinder space 11 which contains a heat exchanger 12 and forms a heat and gas exchange chamber. The heat exchanger 12 is provided with a liquid inlet 13 and a liquid outlet 14. The construction is shown in more detail in Figs. 2 and 3. As is evident from these figures the heat exchanger is made up of a single pipe which consists of two separate parts, namely an inner part 12a and an outer part 12b respectively. Both these parts are coiled in helical form in left-hand twist and right-hand twist respectively. Between the two parts there is a gap 12c. Between it and the outer and inner wall respectively of the vertical cylinder space 11, that is to say the annular heat and gas exchange chamber, the heat exchanger forms further gaps 12d and 12e, respectively.

The gap 10 and the heat and gas exchange chamber 11 formed above it are designed to be located between the centrally arranged gas and liquid inlet pipe 5 and an intermediate vessel 16 which is open at the top.

The gas, which consists of oxygen or an oxygen mixture, when the device is used as an oxygenator, is supplied via an opening 17 and an inlet chamber 18 via a sterile filter (not shown) and further ducts into the annular space 7 in the inlet pipe 6. Through a series of holes 20 which are evenly distributed along the periphery of the gap 10 at the upper end of the latter, the gas is then pressed out into the blood just where the gap 10 passes over into the widened cylinder space 11. Through this arrangement a very effective mixture of blood and gas is achieved. The bubble formation is facilitated, in that the lower part of the space 11 is designed as a mixing chamber which is clear of the heat exchanger. The mixing process is improved further in the following heat exchange 12 with its gaps 12c, 12d, 12e.

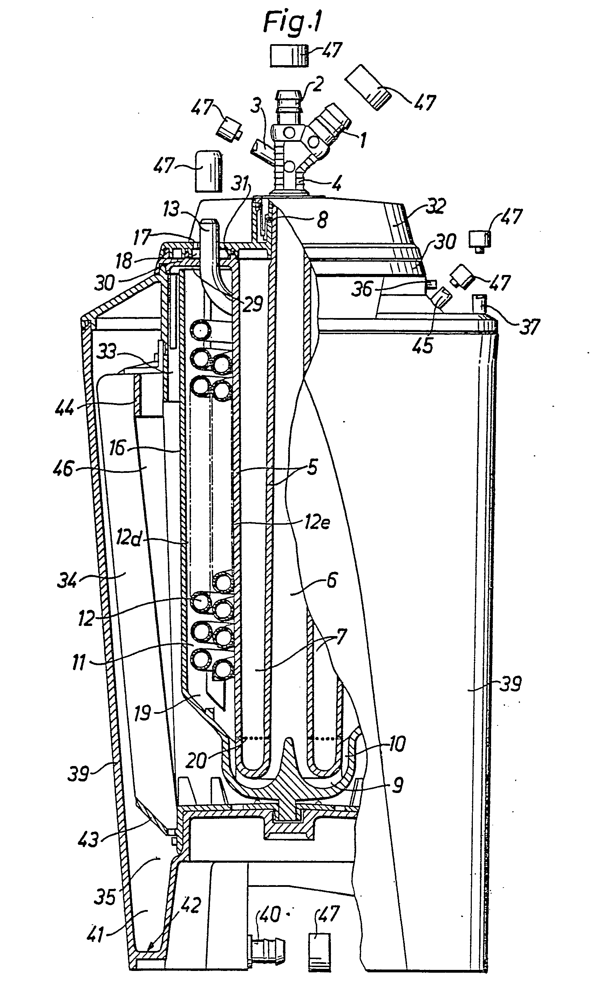

The mixture of gas and liquid thus flows vertically upwards in the said gaps. In the upper end of the heat exchanger an annular throttling gap 29 is shown schematically in Fig. 1 in the form of a spillway between the top edge of the vessel 16 and a lid 30 provided about the same. This lid is designed so as to be integral with the double-walled inlet pipe 5 and is connected in turn via seals 31 to an outer lid 32 comprising, among other things, the gas inlet chamber 18 and the sterile filter (not shown). In accordance with the invention, however, this gap is replaced by the throttling ports 29a shown in Figs. 4—7, which will be described in more detail in connection with these figures.

From the spillway 29 or the throttling ports 29a the gas mixture flows down through a gap 33 between the intermediate vessel 16 and a filter 34. The blood flows out through the filter 34 into blood collecting space 35, whilst the excess gas flows out instead through an outlet 36.

The sampling nozzle 3 is intended for the sampling of venous blood. Via the sampling nozzle 37 and a pipe (not shown) it is possible instead to take samples of arterial blood owing to this pipe terminating down in the blood collecting space 35.

The intermediate vessel 16 with the inlet pipe 5 arranged therein and the filter 34 arranged outside the same, are enclosed in an outer casing 39 comprising a bottom outlet 40 for the separated liquid phase. The lower part of the bottom of this outer casing 39 is designed as an annular gap 41 with a base 42 sloping evenly towards the bottom outlet 40.

It can be said that the construction as a whole is composed of a number of concentric vessels inserted into each other. The outermost of these is the outer casing 39. Into this is inserted a "vessel" formed of the filter 34 which is limited at the bottom by a base ring 43 and at the top by a fixing ring 44. With the help of the ring 44 the filter is fixed to the outside of the vessel 16 described above as the intermediate vessel. Into the latter is then inserted the inlet pipe 5 designed as a double-walled vessel.

The base ring 43 and the fixing ring 44 are designed so that the filter 34 forms an angle against the outer wall of the vessel 16. Through this arrangement a conical space 46 is formed for the collection of the gas and liquid mixture before the same is passed through the filter 34. In this way the blood is made in the first place to pass through the lower part of the filter and to make use only at higher flow also of the upper parts of the filter 34.

Numeral 45 designates an inlet nozzle intended for the supply of medicine, heparin or other additions to the mixture of gas and blood before the same passes the filter 34.

With regard to the holes 20 it may be said that in a preferred embodiment they are constituted of a number of approximately 100 with an accurately defined diameter. This can be made to be practically exactly 25, for example, with the help of a laser technique. With a length of the holes of approximately 3 mm it was found that air will flow through all the holes, even at a gas flow as low as 0.75 litre per minute.

In this preferred embodiment the gap 10 is approximately 3 mm, which provides a good mixture at a standard blood flow of between 2 and 6 litre per minute. However, the device may also be used at higher as well as at lower flow.

Numeral 47 finally designates a number of caps for the closing of different openings of the oxygenator.

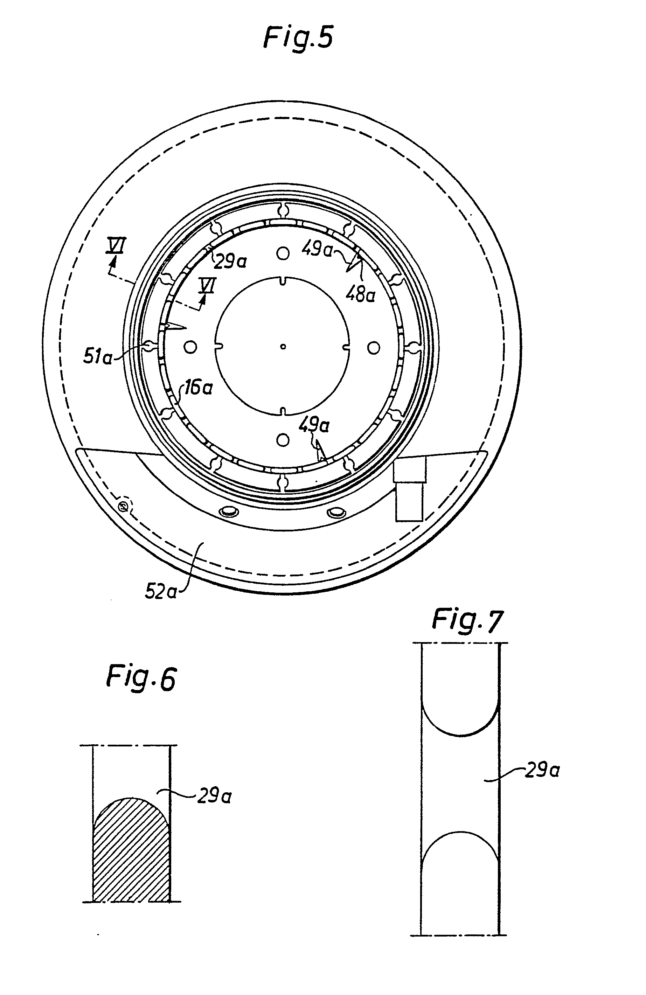

The vessel shown in Figs. 4 and 5 correspond in principle to the vessel 16 in Fig. 1. It has been somewhat modified, however, and for this reason the same reference designations have been used, but with the addition of a small *a*. The vessel is thus designated 16a and the throttling ports, which replace the gap 29, are designated 29a. Numeral 48a designates three ribs which together define the size of the gap, corresponding to the gap 12d formed between the heat exchanger 12 and the vessel 16a. Three shoulders 49a define the lower limit of the heat exchanger or the upper limit of the mixing chamber corresponding to the chamber 19 in Fig. 1. Numeral 50a designates a point which is designated so as to ensure that the blood is deflected smoothly from a flow directed vertically downwards to one directed upwards. The intermediate stage 51a is provided with thickened portions so as to facilitate the outflow of the plastic when the vessel 16a and the lid 52a attached are manufactured by injection moulding.

As is evident from Figs. 6 and 7, the ports 29a are of a double-curved shape. This shape is very important, as it ensures that the blood is handled gently in spite of these ports being subjected to a throttling.

Naturally the invention is not limited to the embodiment described above, but can be varied within the scope of the subsequent claims. The individual shape of the components present, for example, may be varied within wide limits. See also EP—A—0 069 237 submitted at the same time and the above-mentioned EP—A—0 035 266.

**Claims**

1. A device for the transfer of one or more substances between a gas and a liquid, the gas being introduced into the liquid in the form of bubbles, from which the said substances are transferred to the liquid, whereafter the excess gas is removed together with any substances transferred from the liquid to the gas, comprising an annular heat and gas exchange chamber (11) designed for vertical placing and having a gas inlet (20) at the lower end of this chamber and with a heat exchanger (12) arranged above this inlet, wherein the heat and gas exchange chamber (11) is terminated at the top by a plurality of ports (29a) distributed evenly around the periphery, characterized in that the heat and gas exchange chamber (11) is formed between an inner gas and liquid inlet pipe (5) and a vessel (16, 16a) arranged outside the same which is open at the top, the said ports (29a) being constituted of recesses in the top edge of this vessel, which is preferably arranged so as to be in contact with a lid (30) provided over the vessel and the said pipe.

2. A device in accordance with claim 1, characterized in that the ports (29a) are rounded off evenly by a double-curved shape.

3. A device in accordance with claim 1, comprising an outer casing (39) arranged outside the said vessel (16, 16a), which between itself and the vessel forms a skimming chamber (33, 46) with a filter (34) for the final breaking down of the gas bubbles, the filter (34) being arranged obliquely so that between the said vessel and the filter a wedge-shaped collecting space (46) for the liquid widening upwards is formed into which open the said ports (29a).

4. A device in accordance with any one of the preceding claims, characterized in that the heat exchanger (12) is shorter than the heat and gas exchange chamber (11), a mixing chamber (19) being arranged between the gas inlet (20) and the heat exchanger (12).

5. A device in accordance with any one of the preceding claims, characterized in that the gas inlet (20) is constituted of a plurality of holes of substantially the same size distributed evenly around the periphery of the chamber for the formation of bubbles which are partly broken down in a plurality of gaps (12c, 12d, 12e) formed by the heat exchanger (12) in the heat and gas exchange chamber (11), to be broken down further, subsequently, in the said ports (29a).

6. A device in accordance with any one of the preceding claims, characterized in that the heat exchanger (12) is constituted of two pipe parts (12a, 12b) coiled in helical manner, one of which is coiled outside the other, these two parts being separated by a first gap (12c) and forming a second (12d) and a third gap (12e) respectively between themselves and the inner and outer wall respectively of the annular heat and gas exchange chamber (11).

7. A device in accordance with claim 6, characterized in that the two parts (12a, 12b) of the heat exchanger (12) coiled in helical manner constitute parts of one and the same pipe whose ends (13, 14) are threaded through the lid (30) provided above the heat and gas exchange chamber (11).

**Revendications**

1. Dispositif pour effectuer le transfert d'une ou plusieurs substances entre un gaz et un liquide, le gaz étant introduit dans le liquide sous forme de bulles à partir desquelles lesdites substances sont transférées au liquide, après quoi l'excès de gaz est éliminé avec toutes les substances transférées du liquide au gaz, ledit dispositif comprenant une chambre d'échange thermique et gazeux (11) conçue pour être disposée verticalement et comportant une entrée pour le gaz (20) à l'extrémité inférieure de la chambre, avec un èchangeur thermique (12) disposé au-dessus de l'orifice d'entrée, la chambre d'échange thermique et gazeux (11) se terminant à son extrémité supérieure par une pluralité d'orifices (29a) répartis régulièrement autour de sa périphérie, ledit dispositif étant caractérisé en ce que la chambre d'échange thermique et gazeux (11) est formée entre un conduit intérieur d'entrée de gaz et de liquide (5) et un récipient (16, 16a) disposé à l'extérieur du conduit et ouvert à son extrémité supérieure, lesdits orifices (29a) étant constitués par des évidements ménagés dans le bord supérieur du récipient, qui est de préférence agencé de manière à être en contact avec un couvercle (30) disposé sur le récipient et sur ledit conduit.

2. Dispositif suivant la revendication 1, caractérisé en ce que les orifices (29a) sont arrondis par une forme en double-courbe.

3. Dispositif suivant la revendication 1, comprenant un boîtier extérieur (39) disposé à l'extérieur du récipient (16, 16a) qui, entre lui-même et le récipient forme une chambre de séparation (33, 46) avec un filtre (34) pour le fractionnement final des bulles de gaz, le filtre (34) étant disposé obliquement de façon qu'entre le récipient et le filtre soit formé un espace de collecteur en forme d'entonnoir (46) pour le liquide, qui s'évase vers le haut et dans lequel s'ouvrent les orifices (29a).

4. Dispositif suivant l'une quelconque des revendications précédentes, caractérisé en ce que l'échangeur thermique (12) est plus court que la chambre d'échange thermique et gazeux (11), une chambre de mélange (19) étant ménagée entre l'entrée du gaz (30) et l'échangeur thermique (12).

5. Dispositif suivant l'une quelconque des revendications précédentes, caractérisé en ce que l'entrée de gaz (20) est constituée par une pluralité de trous sensiblement de mêmes dimensions répartis régulièrement autour de la périphérie de la chambre pour la formation de bulles qui sont partiellement fracturées dans plusieurs intervalles (12c, 12d, 12e) formés par l'échangeur thermique (12) dans la chambre d'échange thermique et gazeux (11), pour être encore fractionnées ultérieurement dans les orifices précités (29a).

6. Dispositif suivant l'une quelconque des revendications précédentes, caractérisé en ce que l'échangeur thermique (12) est constitué par deux parties de conduit (12a, 12b) enroulées de façon hélicoïdale, dont l'une est enroulée à l'extérieur de l'autre, ces deux parties étant séparées par un premier intervalle (12c) et formant un deuxième (12d) et un troisième (12e) intervalle respectivement, entre elles-mêmes et les parois intérieure et extérieure respectivement de la chambre annulaire d'échange gazeux et thermique.

7. Dispositif suivant la revendication 6, caractérisé en ce que les deux parties (12a, 12b) de l'échangeur thermique (12) enroulé de façon hélicoïdale constituent les parties d'un seul et même conduit dont les extrémités (13, 14) sont enfilées à travers le couvercle (30) disposé sur la chambre d'échange thermique et gazeux (11).

**Patentansprüche**

1. Vorrichtung zur Übertragung einer oder mehrerer Substanzen zwischen einem Gas und einer Flüssigkeit, wobei das Gas in die Flüssigkeit in Blasenform eingeführt wird, aus welcher die Substanzen zu der Flüssigkeit übertragen werden, wonach der Gasüberschuß zusammen mit irgendwelchen aus der Flüssigkeit zum Gas übertragenen Substanzen entfernt wird, mit einer ringförmigen Wärme- und Gasaustauschkammer (11), die für vertikale Anordnung ausgestaltet ist und an ihrem unteren Ende einen Gaseinlaß (20) aufweist, und mit einem Wärmetauscher (12), der über diesem Einlaß angeordnet ist, wobei die Wärme- und Gasaustauscherkammer (11) oben durch eine Vielzahl von Öffnungen (29a) abgeschlossen ist, die gleichmäßig um den Umfang herum verteilt sind, dadurch gekennzeichnet, daß die Wärme- und Gasaustauscherkammer (11) zwischen einer inneren Gas- und Flüssigkeitseinlaßleitung (5) und einem Behälter (16, 16a) gebildet ist, der außerhalb derselben angeordnet ist, die oben offen ist, und daß die Öffnungen (29a) aus Ausnehmungen in der Oberkante dieses Behälters bestehen, der vorzugsweise so angeordnet ist, daß er mit einem Deckel (30), welcher über dem Behälter und der Leitung vorgesehen ist, in Berührung steht.

2. Vorrichtung nach Anspruch 1, dadurch gekennzeichnet, daß die Öffnungen (29a) gleichmäßig durch eine doppelt gekrümmte Gestaltung abgerundet sind.

3. Vorrichtung nach Anspruch 1 mit einem äußeren Gehäuse (39), welches außerhalb des Behälters (16, 16a) angeordnet ist, welches zwischen sich und dem Behälter eine Entschäumungskammer (33, 46) mit einem Filter (34) für das letztliche Aufbrechen der Gasblasen bildet, wobei das Filter (34) schräg so angeordnet ist, daß zwischen dem Behälter und dem Filter ein keilförmiger Sammelraum (46) für die Flüssigkeit, welcher sich nach oben öffnet, gebildet wird, in welchen sich die Öffnungen (29a) hineinöffnen.

4. Vorrichtung nach einem der vorhergehenden Ansprüche, dadurch gekennzeichnet, daß der Wärmetauscher (12) kürzer ist als die Wärme- und Gasaustauscherkammer (11) und daß eine Mischkammer (19) zwischen dem Gaseinlaß (20) und dem Wärmetauscher (12) angeordnet ist.

5. Vorrichtung nach einem der vorhergehenden Ansprüche, dadurch gekennzeichnet, daß der Gaseinlaß (20) aus einer Mehrzahl von Löchern von im wesentlichen gleicher Größe besteht, die gleichmäßig um den Umfang der Kammer herum verteilt sind für die Bildung von Blasen, die teilweise in einer Vielzahl von Spalten (12c, 12d, 12e) aufgebrochen werden, welche durch den Wärmetauscher (12) in der Wärme- und Gasaustauscherkammer (11) gebildet sind, um danach weiterhin in den Öffnungen (29a) aufgebrochen zu werden.

6. Vorrichtung nach einem der vorhergehenden Ansprüche, dadurch gekennzeichnet, daß der Wärmetauscher (12) aus zwei Leitungsteilen (12a, 12b) besteht, die schraubenförmig gewickelt sind, wobei einer derselben außerhalb des anderen herumgewickelt ist, und daß diese zwei Teile durch einen ersten Spalt (12c) getrennt sind sowie einen zweiten (12d) bzw. einen dritten Spalt (12e) zwischen sich und der inneren bzw. äußeren Wand der ringförmigen Wärme- und Gasaustauscherkammer (11) bilden.

7. Vorrichtung nach Anspruch 6, dadurch gekennzeichnet, daß die zwei Teile (12a, 12b) des schraubenförmig gewickelten Wärmetauschers (12) Teile ein und derselben Leitung bilden, deren Enden (13, 14) durch den über der Wärme- und Gasaustauscherkammer (11) vorgesehenen Deckel (30) hindurchgeschraubt sind.

0 0 6 9 2 3 6

Fig.1

## Fig. 2

## Fig. 3

## Fig. 4

Fig.5

Fig.6

Fig.7